# EUROPÄISCHE PATENTANMELDUNG

(11) **EP 0 700 895 A2**
(43) Veröffentlichungstag der Anmeldung: **13.03.1996**
(21) Anmeldenummer: 95112060.9
(22) Anmeldetag: 01.08.1995
(51) Int. Cl.: C07C 227/08, C07C 209/36, C07C 229/56, C07C 211/46

(54) **Verfahren zur selektiven Herstellung von fluorierten aromatischen Aminen und fluorierte aromatische Amine mit besonders niedrigen Gehalten an defluorierten Anteilen**

(30) Priorität: 12.08.1994 DE 4428535
(71) Anmelder: BAYER AG, D-51368 Leverkusen (DE)
(72) Erfinder: Kiel, Wolfgang, Dr., D-51519 Odenthal (DE); Paetz, Klaus-Christian, Dr., D-51399 Burscheid (DE); Müller, Nikolaus, Dr., D-40789 Monheim (DE); Kissener, Wolfram, Dr., D-53819 Neunkirchen-Seelscheid (DE)

(57) **Zusammenfassung**

Fluorierte aromatische Nitroverbindungen werden zu fluorierten aromatischen Aminen katalytisch hydriert, indem man die Hydrierung in flüssiger Phase und in Gegenwart eines Katalysators enthaltend wenigstens ein Metall oder eine Metallverbindung aus der Gruppe Nickel, Kobalt und den Edelmetallen und in Gegenwart mindestens einer Schwefelverbindung der Formel R¹-S(O)ₙ-R² durchführt. So erhaltene fluorierte aromatische Amine sind besonders rein und enthalten besonders wenig defluorierte Produkte.

## Beschreibung

Die vorliegende Erfindung betrifft ein selektives Verfahren zur Herstellung fluorierter aromatischer Amine durch Hydrierung der entsprechenden fluorierten aromatischen Nitroverbindungen und fluorierte aromatische Amine mit besonders niedrigen Gehalten an defluorierten Anteilen.

Bei der Herstellung fluorierter aromatischer Amine durch Hydrierung fluorierter aromatischer Nitroverbindungen ist mit einer mehr oder weniger starken Entfluorierung zu rechnen. Dieser Vorgang ist bei der Hydrierung chlorierter aromatischer Amine, die in großen Mengen durchgeführt wird, allgemein bekannt.

Gerade beim Einsatz fluorhaltiger aromatischer Nitroverbindungen erweist sich bereits eine geringfügige Entfluorierung als äußerst problematisch. Zum einen wirken Fluorid-Ionen auf die Werkstoffe der Produktionsanlagen sehr korrodierend, zum anderen verlangt die Verwendung von fluorierten aromatischen Aminen als Zwischenprodukte für Pharmazeutika und Pflanzenschutzmittel (siehe z.B. EP-A 183 458) möglichst reine, von Nebenkomponenten freie Produkte. Eine nachträgliche Abtrennung geringer Mengen entfluorierter Nebenverbindungen neben einer großen Menge fluorhaltiger Hauptverbindungen wäre wegen der geringen Unterschiede in ihren physikalischen Eigenschaften sehr aufwendig und deshalb unter wirtschaftlichen Aspekten nicht zu vertreten.

Es ist somit gerade bei der Herstellung von fluorierten aromatischen Aminen ein dringendes Bedürfnis vorhanden, die Fluorabspaltung weitestgehend zu unterbinden.

Es wurde nun ein Verfahren zur katalytischen Hydrierung von fluorierten aromatischen Nitroverbindungen zu fluorierten aromatischen Aminen in Gegenwart von Katalysatoren gefunden, das dadurch gekennzeichnet ist, daß man die Hydrierung in flüssiger Phase und in Gegenwart eines Katalysators enthaltend wenigstens ein Metall oder eine Metallverbindung aus der Gruppe Nickel, Kobalt und die Edelmetalle und in Gegenwart mindestens einer Schwefelverbindung der Formel (I) durchführt

R¹-S(O)ₙ-R² (I),

in der
- R¹ und R²: unabhängig voneinander jeweils geradkettiges oder verzweigtes C₁-C₁₂-Alkyl, Hydroxy-C₁-C₁₂-alkyl, Carboxy-C₁-C₁₂-alkyl oder Phenyl bedeuten,
- R¹: zusätzlich Wasserstoff oder CO-C₁-C₁₂-Alkyl bedeuten kann,
- R¹ und R²: gemeinsam auch -CH=CH-CH=CH-, -(CH₂)₄-, -(CH₂)₅- oder -(CH₂)₂-X-(CH₂)₂- mit X = Sauerstoff oder Schwefel bedeuten können und
- n: für Null oder 1 steht.

In das erfindungsgemäße Verfahren kann man z.B. fluorierte aromatische Nitroverbindungen der Formel (II) einsetzen 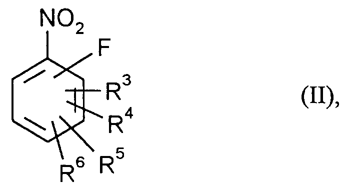
in der
- R³, R⁴, R⁵ und R⁶: unabhängig voneinander jeweils H, COOH, Halogen, NH₂, NO₂, OH, CCl₃, CF₃ oder gegebenenfalls mit 1 bis 2 Hydroxy-, Amino-, Nitril- oder Carboxyl-Gruppen substituiertes C₁-C₁₀-Alkyl, C₆-C₁₀-Aryl, C₇-C₁₄-Aralkyl oder C₆-C₁₀-Arylsulfonyl bedeuten
und die entsprechenden fluorierten aromatischen Amine der Formel (III) erhalten 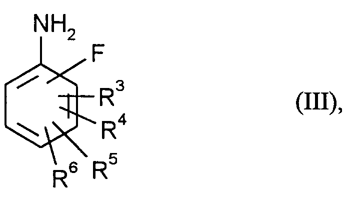
in der
- R³, R⁴, R⁵ und R⁶: die bei Formel (II) angegebene Bedeutung haben.

Halogen steht bei den Formeln (II) und (III) vorzugsweise für Fluor, Chlor oder Brom.

Vorzugsweise setzt man in das erfindungsgemäße Verfahren fluorierte aromatische Nitroverbindungen der Formel (II) ein, bei denen R³ bis R⁶, R³ bis R⁵ oder R³ und R⁴ Wasserstoff und die verbleibenden Reste aus der Gruppe R³ bis R⁶ unabhängig voneinander jeweils COOH, F, Cl, NH₂, NO₂, OH, CCl₃ oder CF₃ bedeuten.

Besonders bevorzugte Verbindungen der Formel (II) sind: 5-Fluor-2-nitro-benzoesäure, 4-Fluor-3-nitro-benzoesäure, 3-Fluor-2-nitro-benzoesäure, o-Fluornitrobenzol, n-Fluornitrobenzol, p-Fluornitrobenzol, 4-Fluor-2-chlor-nitrobenzol, 2-Fluor-5-chlor-nitrobenzol, 4-Fluor-2-trichlormethyl-nitrobenzol, 4-Fluor-2-trifluormethyl-nitrobenzol, 2,4-Dinitro-fluorbenzol, 2,5-Difluor-nitrobenzol, 2-Fluor-5-hydroxy-nitrobenzol und 2-Amino-4-fluor-nitrobenzol.

Zum Einsatz in das erfindungsgemäße Verfahren besonders geeignete 5-Fluor-2-nitro-benzoesäure kann man beispielsweise gemäß Recl. Trav. Chim. Pays-Bas 33, 336 (1914), Liebigs Ann. Chem. 1976, 946-968, Tetrahedron Lett 40, 5105 bis 5108 (1988) oder auf folgende Weise erhalten: 3-Fluorbenzoesäure wird in 100 %iger Schwefelsäure gelöst und zu dieser Lösung Mischsäure (67 % Schwefelsäure/33 % Salpetersäure) bei 5 bis 20°C im Verlauf von 2 bis 10 Stunden zugetropft. Anschließend wird bei der gleichen Temperatur 30 Minuten bis 2 Stunden nachgerührt. Dann wird das Reaktionsgemisch auf Wasser ausgetragen, wobei man eine Temperatur von 60°C nicht überschreitet. Es wird auf Raumtemperatur abgekühlt, filtriert, der Filterkuchen mit Wasser gewaschen und getrocknet.

Bevorzugte Schwefelverbindungen der Formel (I) sind solche, bei denen R¹ und R² gleich sind und Hydroxy-C₁-C₆-alkyl bedeuten. Ganz besonders bevorzugt ist Bis-(2-Hydroxyethyl)-sulfid.

Als Edelmetalle für den Katalysator kommen z.B. Ruthenium, Rhodium, Palladium, Osmium, Iridium und Platin in Frage. Bevorzugt sind Palladium und Platin, besonders bevorzugt ist Platin. Nickel, Cobalt, Edelmetalle oder deren Verbindungen können gegebenenfalls auf einem Träger vorliegen. Ein bevorzugtes Trägermaterial ist Kohle.

Die Herstellung eines aus Edelmetall auf Kohle bestehenden Katalysators, der für das erfindungsgemäße Verfahren eingesetzt werden kann, kann beispielsweise so erfolgen, daß man stückige Kohle in der wäßrigen Lösung einer Edelmetallverbindung aufschlämmt und dann das Edelmetall durch Zugabe eines Reduktionsmittels, z.B. Wasserstoff oder Hydrazin, auf der Kohle ausfällt.

An die Beschaffenheit der Kohle werden keine besonderen Anforderungen gestellt. Bevorzugt sind Kohlen mit einer wirksamen Oberfläche von mindestens 800 m²/g.

Bevorzugt werden Edelmetalle auf Kohle-Katalysatoren verwendet, die 0,5 bis 5 Gew.-% Edelmetall enthalten.

Die Menge des Katalysators ist nicht kritisch und kann in weiten Grenzen variiert werden. Beispielsweise kann man 0,02 bis 3 Gew.-%, bevorzugt 0,1 bis 1 Gew.-% Edelmetall-Katalysator, bezogen auf die Verbindung der Formel (II) einsetzen.

Das Gewichtsverhältnis der eingesetzten Schwefelverbindungen der Formel (I) zu Katalysator kann z.B. 0,001 bis 0,125:1 betragen. Bevorzugt liegt es bei 0,0025 bis 0,025:1, ganz besonders bevorzugt bei 0,005 bis 0,0125:1.

Die Schwefelverbindungen der Formel (I) können als einzelne Verbindungen oder als Mischung verschiedener Einzelverbindungen eingesetzt werden. Vorteilhafterweise werden die Schwefelverbindungen in Form von Lösungen eingesetzt. Beispielsweise können sie in 0,01 bis 1 gew.-%iger wäßriger oder toluolischer Lösung zum Reaktionsgemisch gegeben werden. Man kann die Schwefelverbindungen auch vor der Reaktion dem Katalysator zumischen.

Für das erfindungsgemäße Verfahren ist es im allgemeinen nur beim ersten Einsatz eines Edelmetallkatalysators erforderlich, Schwefelverbindungen zuzusetzen. Die Katalysatoren behalten häufig auch nach vielfach wiederholter Anwendung und auch bei kontinuierlicher Arbeitsweise über lange Zeit ihre Aktivität und Selektivität, ohne daß laufend neue Schwefelverbindungen zugegeben werden müssen. Wenn sich ein allmähliches Nachlassen der Selektivität zeigt, kann die ursprüngliche Selektivität durch erneute Zugabe von Schwefelverbindungen wieder hergestellt werden. Bei einer derartigen erneuten Zugabe von Schwefelverbindungen sind häufig 0,5 bis 15 Gew.-% der ursprünglich eingesetzten Menge ausreichend.

Das erfindungsgemäße Verfahren wird in flüssiger Phase durchgeführt. Als Lösungsmittel kommen unter den Reaktionsbedingungen inerte Lösungsmittel in Betracht, beispielsweise Wasser, Alkohole mit 1 bis 6 C-Atomen und aromatische Kohlenwasserstoffe mit 6 bis 10 C-Atomen. Bevorzugt sind Methanol, Isopropanol, Benzol, Toluol und Xylole, besonders bevorzugt Methanol und Toluol.

Als Katalysatoren können auch Nickel und/oder Kobalt enthaltende eingesetzt werden wie elementares Nickel auf Trägern, elementares Nickel in Form von Nickelschwamm, Nickeloxid, Raney-Nickel oder die entsprechenden Kobalt-Katalysatoren. Trägermaterialien, auch für Edelmetallkatalysatoren, können außer Kohle beispielsweise Siliciumdioxide, Aluminiumoxide, Bims und andere dem Fachmann bekannte Träger sein. Bevorzugt sind von den Nicht-Edelmetall-Katalysatoren Raney-Katalysatoren wie Raney-Nickel, Raney-Kobalt, Raney-Nickel-Eisen, Raney-Nickel-Kobalt und Raney-Nickel-Eisen-Kobalt, die z.B. in wasserfeuchter oder lösungsmittelfeuchter Form eingesetzt werden können. Bei Nickel- oder Kobalt-haltigen Katalysatoren kann ein Zusatz größerer Mengen an Schwefelverbindungen der Formel (I) als bei Edelmetallkatalysatoren günstig sein. Bevorzugt setzt man 0,01 bis 0,1 Gew.-Teile Schwefelverbindungen der Formel (I) je Gewichtsteil Nickel- und/oder Kobalt-enthaltenden Katalysator ein. Im übrigen kann mit den Nickel- und/oder Kobalt-enthaltenden Katalysatoren wie bei den Edelmetall-Katalysatoren näher beschrieben verfahren werden.

Die Hydrierung kann beispielsweise bei 30 bis 250°C, bevorzugt bei 50 bis 140°C, und einem H₂-Druck von 3 bis 300 bar, bevorzugt 10 bis 150 bar, durchgeführt werden.

Bei einer bevorzugten Variante des erfindungsgemäßen Verfahrens werden Fluornitro-benzoesäuren bei einem pH-Wert von 8 bis 14, bevorzugt 9 bis 11 katalytisch hydriert. Solche pH-Werte kann man durch Zusatz von basischen Stoffen einstellen, beispielsweise mit Natriumhydroxid, Kaliumhydroxid, Calciumhydroxid (z.B. in der Form von gelöschtem Kalk), Calciumoxid, Kaliumcarbonat oder Natriumcarbonat. Die basischen Stoffe können, soweit sie wasserlöslich sind, als wäßrige Lösung, oder in Substanz eingesetzt werden. Bevorzugt wird wäßrige Natronlauge eingesetzt.

Überraschenderweise wird die Fluorabspaltung beim erfindungsgemäßen Verfahren nicht gefördert, wenn man basische Stoffe zusetzt.

Man kann das erfindungsgemäße Verfahren z.B. so durchführen, daß man die fluorierte aromatische Nitroverbindung der Formel (II), das Lösungsmittel, den Katalysator, die Schwefelverbindung der Formel (I) und gegebenenfalls die Base in einem Hydrierautoklaven vorlegt und nach dem Verschließen des Reaktors die Luft mit Stickstoff und anschließend den Stickstoff mit Wasserstoff verdrängt. Nach beendeter Reaktion wird das Reaktionsgefäß im allgemeinen zunächst entspannt und entleert. Aus dem Reaktionsgemisch kann man den Katalysator durch Abfiltrieren abtrennen und ihn ohne neuen Zusatz von Schwefelverbindungen wiederverwenden.

Aus dem bei der Katalysatorabtrennung anfallenden Filtrat kann man dann das hergestellte fluorierte aromatische Amin isolieren.

Das erfindungsgemäße Verfahren kann sowohl diskontinuierlich als auch kontinuierlich durchgeführt werden.

Im Falle der Herstellung fluorierter Aminobenzoesäuren kann man diese nach dem Abtrennen des Katalysators z.B. durch Ansäuern, z.B. auf einen pH-Wert von 3 bis 4, freisetzen und durch Filtration oder Extraktion in reiner Form gewinnen.

Mit dem erfindungsgemäßen Verfahren gelingt es überraschenderweise fluorierte aromatische Amine zu erhalten, die einen Reinheitsgrad von über 96 %, häufig über 99 % aufweisen und die weniger als 0,1, häufig weniger als 0,01 Gew.-% defluoriertes Produkt enthalten. Insbesondere kann man nach dem erfindungsgemäßen Verfahren 5-Fluor-anthranilsäure (= 5-Fluor-2-amino-benzoesäure) mit einem Reinheitsgrad von über 99 % und einem Gehalt an defluoriertem Produkt von weniger als 0,01 % erhalten.

Die vorliegende Erfindung betrifft deshalb auch fluorierte aromatische Amine der Formel (III) mit einem Reinheitsgrad von über 96 % und einem Gehalt an defluorierten Produkten von weniger als 0,1 %. Weiterhin betrifft die vorliegende Erfindung 5-Fluor-anthranilsäure mit einem Reinheitsgrad von über 99 % und einem Gehalt an defluorierten Produkten von weniger als 0,01 %.

### Beispiele

Prozente und Teile beziehen sich hier wie sonst im Text auf das Gewicht, soweit nichts anderes angegeben ist.

### Allgemeine Arbeitsweise:

In einem Autoklaven wurden die fluorierte aromatische Nitroverbindung, das Lösungsmittel, der Katalysator und die gegebenenfalls in Wasser oder einem organischen Lösungsmittel gelöste Schwefelverbindung vorgelegt. Der Autoklav wurde zuerst mit Stickstoff und dann mit Wasserstoff gespült. Anschließend wurde auf die angegebene Temperatur erhitzt und bei dem jeweils angeführten Wasserstoff-Druck hydriert, bis keine Wasserstoff-Aufnahme mehr erfolgte. Nach Beendigung der Reaktion wurde der Katalysator abfiltriert und für weitere Hydrierungen bereitgestellt. Aus dem Filtrat wurde durch Fällen durch Ansäuern und Filtration oder Destillation das fluorierte aromatische Amin isoliert. Die Gehalte an defluoriertem Produkt und an gewünschtem fluoriertem aromatischem Amin wurden mittels Gaschromatographie bestimmt.

Nach dieser allgemeinen Vorschrift wurden folgende Beispiele durchgeführt:

### Beispiele 1 bis 3

### Hydrierung von 5-Fluor-2-nitro-benzoesäure

Ein Gemisch aus 82 g 5-Fluor-2-nitro-benzoesäure, 2,5 g Platin-auf-Kohle (1 %ig), die jeweils angegebene Menge Bis-(2-hydroxyethyl)-sulfid (BHS) und 300 ml Wasser wurden im Autoklaven mit verdünnter Natronlauge auf einen pH-Wert von 10 eingestellt und bei 50 bis 60°C und 80 bar Wasserstoffdruck hydriert. Nach dem Abfiltrieren des Katalysators wurde das Filtrat mit verdünnter Schwefelsäure auf einen pH-Wert von 4 eingestellt und die ausgefallene 5-Fluor-anthranilsäure abfiltriert. Die Menge an zugesetzter Schwefelverbindung ist hier und in den anderen Beispielen in Prozent bezogen auf den Katalysator angegeben. Für Einzelheiten siehe folgende Tabelle.

**Tabelle**

| (für Beispiele 1 bis 3) | | | | |
|---|---|---|---|---|
| | | | Reaktionsprodukt | |
| Bsp. Nr. | BHS (%) | Reaktionszeit (min) | Gehalt an defluoriertem Produkt (%) | Gehalt an 5-Fluoranthranilsäure (%) |
| 1* | - | 20 | 1,22 | >98 |
| 2 | 0,3 | 20 | 0,008 | >99 |
| 3 | 0,6 | 25 | 0,002 | >99 |

| | | | | |
|---|---|---|---|---|
| *) Vergleichsversuch ohne BHS-Zusatz | | | | |

### Beispiele 4 bis 7

Beispiel 3 wurde viermal wiederholt, wobei stets der beim vorhergehenden Beispiel abgetrennte Katalysator eingesetzt und kein weiteres BHS zugesetzt wurde. Für Einzelheiten siehe die folgende Tabelle.

**Tabelle**

| (für Beispiele 4 bis 7) | | | |
|---|---|---|---|
| | | Reaktionsprodukt | |
| Bsp. Nr. | Reaktionszeit (min) | Gehalt an defluoriertem Produkt (%) | Gehalt an 5-Fluoranthranilsäure (%) |
| 4 | 30 | 0,002 | >99 |
| 5 | 30 | 0,003 | >99 |
| 6 | 25 | 0,003 | >99 |
| 7 | 30 | 0,004 | >99 |

### Beispiel 8

Beispiel 3 wurde wiederholt, wobei der beim Beispiel 3 abgetrennte Katalysator und 1 % BHS zusätzlich eingesetzt wurden. Nach einer Reaktionszeit von 45 Minuten wurde über 99 % reine 5-Fluoranthranilsäure mit einem Gehalt an defluoriertem Produkt von unter 0,001 % erhalten.

### Beispiele 9 bis 14

### Hydrierung von 4-Nitrofluorbenzol

36 g 4-Nitrofluorbenzol, 3 g des jeweils angegebenen Katalysators, die jeweils angegebene Menge BHS und 194 g Methanol wurden gemischt und analog den Beispielen 1 bis 3 bei 100°C und 60 bar Wasserstoffdruck hydriert.

Für Einzelheiten siehe die folgende Tabelle.

**Tabelle**

| (für Beispiele 9 bis 14) | | | | | |
|---|---|---|---|---|---|
| | | | | Reaktionsprodukt | |
| Bsp. Nr. | Katalysator | BHS (%) | Reaktionszeit (min) | Gehalt an defluoriertem Produkt (%) | Gehalt an 4-Fluoranilin (%) |
| 9* | Raney-Nickel | - | 15 | 7,86 | 91,08 |
| 10 | Raney-Nickel | 10 | 9 | 0,07 | 96,38 |
| 11* | Pt/Kohle 1%ig | - | 2 | 2,51 | 96,15 |
| 12 | Pt/Kohle 1%ig | 1 | 9 | 0,04 | 99,30 |
| 13* | Pt/Kohle 3%ig, sulfitiert | - | 11 | 0,12 | 98,4 |
| 14 | Pt/Kohle 3%ig, sulfitiert | 1 | 10 | <0,01 | 99,5 |

| | | | | | |
|---|---|---|---|---|---|
| *Vergleichsversuch ohne BHS-Zusatz | | | | | |

## Patentansprüche

1. Verfahren zur katalytischen Hydrierung von fluorierten aromatischen Nitroverbindungen in Gegenwart von Katalysatoren, dadurch gekennzeichnet, daß man die Hydrierung in flüssiger Phase und in Gegenwart eines Katalysators enthaltend wenigstens ein Metall oder eine Metallverbindung aus der Gruppe Nickel, Kobalt und die Edelmetalle und in Gegenwart mindestens einer Schwefelverbindung der Formel (I) durchführt
R¹-S(O)ₙ-R² (I),
in der
R¹ und R² unabhängig voneinander jeweils geradkettiges oder verzweigtes C₁-C₁₂-Alkyl, Hydroxy-C₁-C₁₂-alkyl, Carboxy-C₁-C₁₂-alkyl oder Phenyl bedeuten,
R¹ zusätzlich Wasserstoff oder CO-C₁-C₁₂-Alkyl bedeuten kann,
R¹ und R² gemeinsam auch -CH=CH-CH=CH-, -(CH₂)₄-, -(CH₂)₅- oder -(CH₂)₂-X-(CH₂)₂- mit X = Sauerstoff oder Schwefel bedeuten können und
n für Null oder 1 steht.

2. Verfahren nach Anspruch 1, dadurch gekennzeichnet, daß man fluorierte aromatische Nitroverbindungen der Formel (II) einsetzt 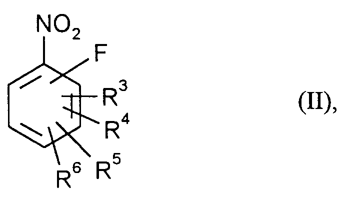 in der
R³, R⁴, R⁵ und R⁶ unabhängig voneinander jeweils H, COOH, Halogen, NH₂, NO₂, OH, CCl₃, CF₃ oder gegebenenfalls mit 1 bis 2 Hydroxy-, Amino-, Nitril- oder Carboxyl-Gruppen substituiertes C₁-C₁₀-Alkyl, C₆-C₁₀-Aryl, C₇-C₁₄-Aralkyl oder C₆-C₁₀-Arylsulfonyl bedeuten
und die entsprechenden fluorierten aromatischen Amine der Formel (III) erhält 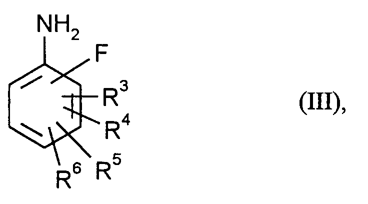 in der
R³, R⁴, R⁵ und R⁶ die bei Formel (II) angegebene Bedeutung haben.

3. Verfahren nach Anspruch 2, dadurch gekennzeichnet, daß man Verbindungen der Formel (II) einsetzt, bei denen R³ bis R⁶, R³ bis R⁵ oder R³ und R⁴ Wasserstoff und die verbleibenden Reste aus der Gruppe R³ bis R⁶ unabhängig voneinander jeweils COOH, F, Cl, NH₂, NO₂, OH, CCl₃ oder CF₃ bedeuten.

4. Verfahren nach Ansprüchen 1 bis 3, dadurch gekennzeichnet, daß man 5-Fluor-2-nitro-benzoesäure einsetzt, die erhalten wurde durch Auflösen von 3-Fluorbenzoesäure in 100 %iger Schwefelsäure, Zutropfen lassen von Mischsäure (67 % Schwefelsäure / 33 % Salpetersäure) bei 5 bis 20°C im Verlauf von ca. 2 bis 10 Stunden, Nachrühren bei dieser Temperatur für 30 Minuten bis 2 Stunden, Austragen des Reaktionsgemisches auf Wasser, wobei 60°C nicht überschritten werden, Abkühlung auf Raumtemperatur, Filtration, Waschen des Filterkuchens mit Wasser und Trockung.

5. Verfahren nach Ansprüchen 1 bis 4, dadurch gekennzeichnet, daß in Formel (I) R¹ und R² gleich sind und Hydroxy-C₁-C₆-alkyl bedeuten.

6. Verfahren nach Ansprüchen 1 bis 5, dadurch gekennzeichnet, daß der Katalysator Ruthenium, Rhodium, Palladium, Osmium, Iridium, Platin, Nickel oder Kobalt enthält.

7. Verfahren nach Ansprüchen 2 bis 6, dadurch gekennzeichnet, daß, bezogen auf die Verbindung der Formel (II), 0,02 bis 3 Gew.-% Edelmetallkatalysator und, bezogen auf den Katalysator, Schwefelverbindungen der Formel (I) in Gewichtsmengen von 0,001 bis 0,125:1 und Wasser, Alkohole mit 1 bis 6 C-Atomen oder aromatische Kohlenwasserstoffe mit 6 bis 10 C-Atomen als Lösungsmittel einsetzt.

8. Verfahren nach Ansprüchen 1 bis 7, dadurch gekennzeichnet, daß man es bei 30 bis 250°C und einem H₂-Druck von 3 bis 300 bar durchführt.

9. Fluorierte aromatische Amine der Formel (III) 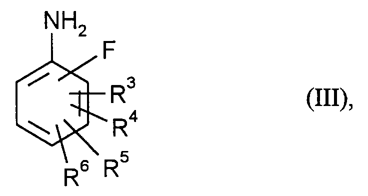 in der
R³, R⁴, R⁵ und R⁶ die Anspruch 2 angegebene Bedeutung haben,
mit einem Reinheitsgrad von über 96 % und einem Gehalt an defluorierten Produkten von weniger als 0,1 %.

10. 5-Fluoranthranilsäure mit einem Reinheitsgrad von über 99 % und einem Gehalt an defluorierten Produkten von weniger als 0,01 %.
